# EUROPEAN PATENT APPLICATION

(11) **EP 3 403 680 A1**
(43) Date of publication of application: **21.11.2018**
(21) Application number: 17741528.8
(22) Date of filing: 20.01.2017
(51) Int. Cl.: A61M 5/145

(54) **SYRINGE AND SYRINGE PUMP DEVICE INCLUDING SAME**

(30) Priority: 21.01.2016 JP 2016009342
(71) Applicant: Daiken Medical Co., Ltd., Osaka 541-0045 (JP)
(72) Inventor: MIYAMURA, Yukiharu, Izumi-shi Osaka 594-1157 (JP)
(74) Representative: TBK
(86) International application number: PCT/JP2017/001920
(87) International publication number: WO 2017/126651

(57) **Abstract**

A syringe (4) includes a cylinder body (42) having a second axial end provided with a support (45), a plunger (43) configured to axially reciprocate inside the cylinder body (42), and an operation part (44) allowing the plunger (43) to be inserted therethrough, and configured to be rotatable about an axis of the plunger (43). The plunger (43) has an outer circumferential surface provided with a male screw (431) and a plunger groove (432). The operation part (44) has an inner circumferential surface provided with a female screw (441) to be screwed to the male screw (431), and includes an operation insert (443) having an outer circumferential surface provided with an operation groove (443A). The support (45) of the cylinder body (42) is provided with an operation part shift restrictor (451) that is inserted to the operation groove (443A) and restricts a shift of the operation part (44), and a plunger rotation restrictor (452) that is inserted to the plunger groove (432) and restricts rotation of the plunger (43).

## Description

### Technical Field

The present invention relates to a syringe configured to suck or discharge fluid, and a syringe pump device including the syringe.

### Background Art

Patent Literature 1 or the like discloses a syringe pump device used in the medical field and the like and configured to precisely suck or discharge fluid. The syringe pump device according to Patent Literature 1 includes a device body detachably holding a syringe containing fluid, and a slider feed mechanism. The syringe pump device includes a syringe used typically in the medical field and the like and including a cylinder containing fluid and a plunger configured to axially reciprocate in the cylinder. In a state where the syringe thus configured is attached to the device body in the syringe pump device, the slider feed mechanism axially pushes the plunger of the syringe to discharge the fluid in the cylinder.

The syringe pump device disclosed in Patent Literature 1 causes the syringe typically used in the medical field and the like to suck or discharge the fluid, and thus has limitation in precise control of a flow rate of the sucked or discharged fluid. If the plunger of the syringe receives unintended axial force of an operator of the syringe pump device, the plunger is likely to be shifted axially to cause undesired suck or discharge of the fluid into or from the cylinder.

### Citation List

### Patent Literature

Patent Literature 1: JPH9-122237 A

### Summary of Invention

It is an object of the present invention to provide a syringe configured to suck or discharge fluid, which is possible to precisely control a flow rate of the sucked or discharged fluid, and restrain undesired suck or discharged of the fluid, as well as provide a syringe pump device including the syringe.

A syringe according to an aspect of the present invention includes: a cylinder including a cylinder body having an internal space to accommodate fluid, and a fluid flow-through channel part connected to a first axial end of the cylinder body and provided with a fluid flow-through channel communicating with the internal space; a plunger configured to axially reciprocate inside the cylinder body; an operation part having a tubular shape allowing the plunger to be inserted therethrough, and supported at a second axial end opposite to the first axial end of the cylinder body to be rotatable about an axis of the plunger relative to the cylinder body; and a converter configured to convert torque of the operation part to axially reciprocating force of the plunger, and to restrict a shift of the plunger in an axial direction due to axial force applied the plunger.

A syringe pump device according to another aspect of the present invention includes: the syringe described above; a drive source configured to generate drive power causing rotation of the operation part in the syringe; a drive power transfer part configured to transfer the drive power generated by the drive source to the gear part of the operation part; and a holder holding the syringe, the drive source, and the drive power transfer part.

The present invention provides such a syringe configured to suck or discharge fluid, which is possible to precisely control a flow rate of the sucked or discharged fluid, and restrain undesired suck or discharged of the fluid, as well as provides a syringe pump device including the syringe.

The object, features, and advantages of the present invention will be made clearer with the following detailed description and accompanying drawings.

### Brief Description of Drawings

FIG. 1 is a perspective view schematically showing an entire configuration of a syringe pump device according to an embodiment of the present invention.
FIG. 2 is a sectional view in a direction of an arrow X1, of the syringe pump device shown in FIG. 1.
FIG. 3 is a sectional view in a direction of an arrow X2, of the syringe pump device shown in FIG. 1.
FIG. 4 is a perspective view showing a configuration of a syringe according to an embodiment of the present invention.
FIG. 5A is a plan view of the syringe shown in FIG. 4.
FIG. 5B is a side view of the syringe shown in FIG. 4.
FIG. 6A is a sectional view along a sectional line VI-VI, of the syringe shown in FIG. 5A.
FIG. 6B is a sectional view along the sectional line VI-VI, of part of the syringe shown in FIG. 5A.
FIG. 7A is a sectional view along a sectional line VII-VII, of the syringe shown in FIG. 5B.
FIG. 7B is a sectional view along the sectional line VII-VII, of part of the syringe shown in FIG. 5B.

### Description of Embodiments

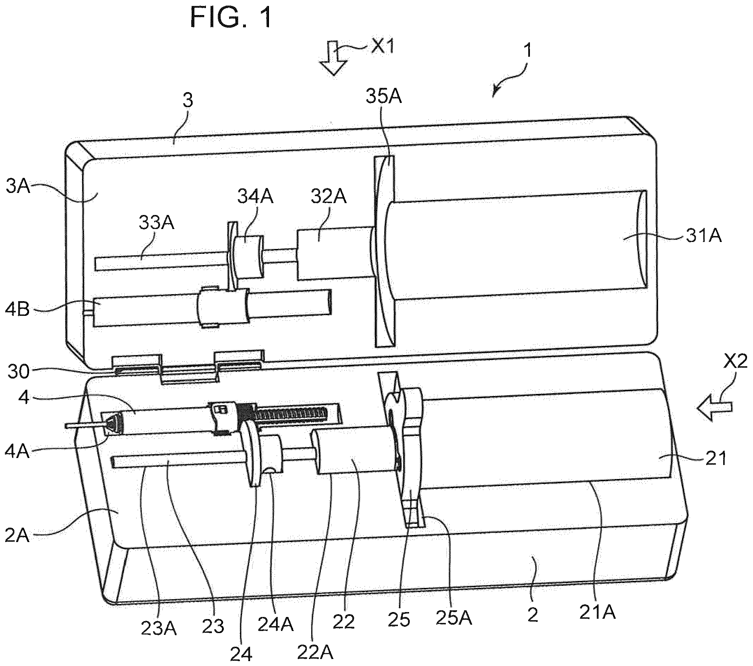

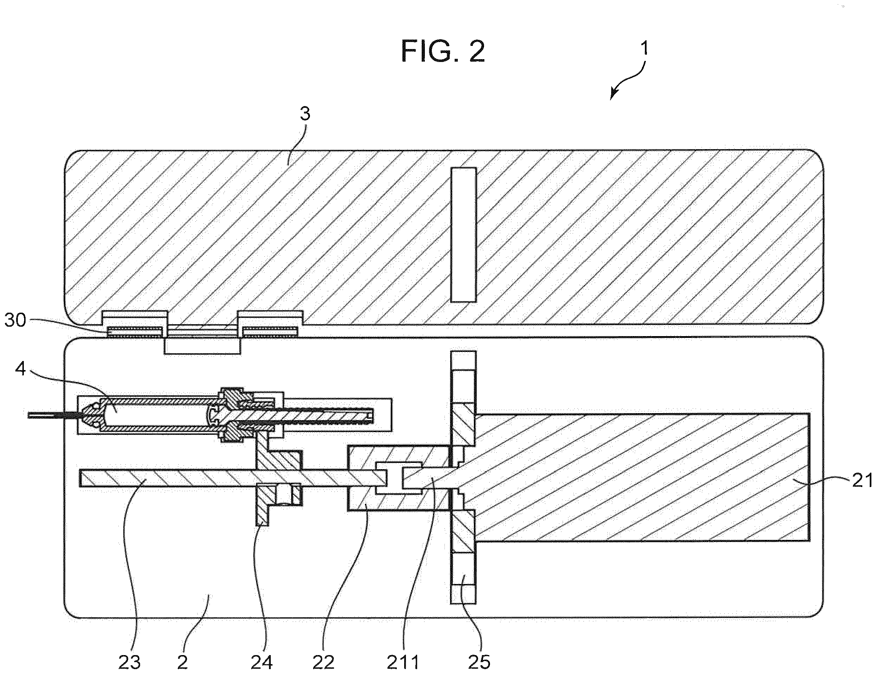

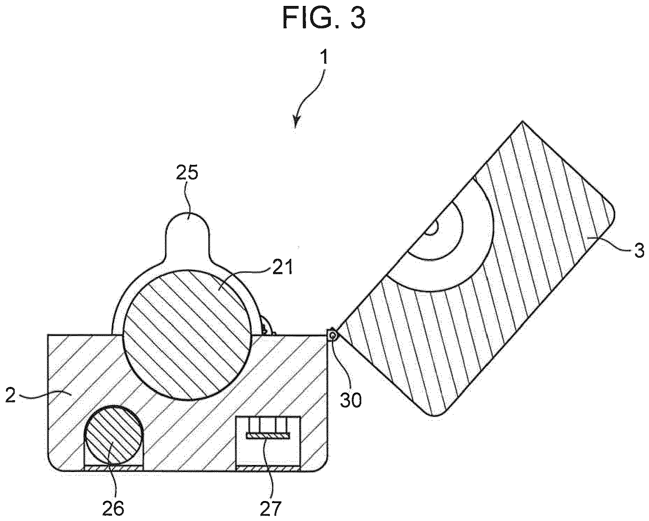

A syringe and a syringe pump device according to an embodiment of the present invention will now be described with reference to drawings. The embodiment to be described below exemplarily implements the present invention, and should not limit the technical scope of the present invention. FIG. 1 is a perspective view schematically showing an entire configuration of a syringe pump device 1 according to an embodiment of the present invention. FIG. 2 is a sectional view in a direction of an arrow X1, of the syringe pump device 1 shown in FIG. 1. FIG. 3 is a sectional view in a direction of an arrow X2, of the syringe pump device 1 shown in FIG. 1.

The syringe pump device 1 according to the present embodiment is configured to suck or discharge fluid used in the medical field and the like. Specifically, the syringe pump device 1 is used to suck or discharge liquid medicine as fluid for genetic testing and the like. Such genetic testing is executed to analyze nucleic acids, chromosomes, and the like and clinically check variations, karyotypes, and the like relating hereditary diseases. Examples of the genetic testing include determination of whether or not a specimen collected from a living body contains any nucleic acid (target nucleic acid) derived from infection causative bacteria as pathogenic bacteria of infectious diseases like tuberculosis. The genetic testing needs process liquid for preprocessing of the specimen, exemplified by mixed liquor of NALC (N-acetyl-L-cysteine)-NaOH (sodium hydroxide), TB-Beads (registered trademark) solution (manufactured by Japan BCG Laboratory), and an elution buffer of TB-Beads (registered trademark) (manufactured by Japan BCG Laboratory). The genetic testing also needs a reaction reagent for nucleic acid amplification reaction of the target nucleic acid. Such a reaction reagent includes a primer to be bonded to the target nucleic acid, and an enzyme serving as a catalyst for the nucleic acid amplification reaction. The syringe pump device 1 is used to suck or discharge fluid such as the process liquid or the reaction reagent. The syringe pump device 1 is also used for nutriment feeding, blood transfusion, or infusion of liquid medicine such as a chemotherapeutic agent or an anesthetic.

The syringe pump device 1 according to the present embodiment includes a device body 2, a lid 3, a syringe 4, a drive motor 21 (drive body), an output shaft 211, a coupling 22, a drive shaft 23, a drive gear 24, an encoder 25, a power source 26, and a controller 27. The present embodiment provides a drive source configured by the drive motor 21 and the output shaft 211, and a drive power transfer part configured by the coupling 22, the drive shaft 23, and the drive gear 24. The device body 2 and the lid 3 configure a holder of the syringe 4, the drive source, and the drive power transfer part.

The device body 2 has a structure provided with components of the syringe pump device 1. The device body 2 is not particularly limited in terms of a shape thereof. The device body 2 according to the present embodiment has a rectangular parallelepiped shape with height smaller than longitudinal length and width. The device body 2 has a principal surface 2A provided with a first syringe disposing recess 4A as a region for the syringe 4, a first body recess 21A as a region for the drive motor 21, a second body recess 22A as a region for the coupling 22, a third body recess 23A as a region for the drive shaft 23, a fourth body recess 24A as a region for the drive gear 24, and a fifth body recess 25A as a region for the encoder 25.

The lid 3 is openably connected to the device body 2 via an open/close connection 30 provided at an outer peripheral edge of the lid 3, to cover the device body 2 from above when closed. When the lid 3 covers to close the device body 2 from above, the syringe 4, the drive source, and the drive power transfer part as the components of the syringe pump device 1 are interposed to be held between the device body 2 and the lid 3. The lid 3 has a rectangular parallelepiped shape with height smaller than longitudinal length and width, to match the shape of the device body 2. The lid 3 has an opposing surface 3A facing the principal surface 2A of the device body 2 in the state where the lid 3 covers to close the device body 2 from above. The opposing surface 3A of the lid 3 is provided with a second syringe disposing recess 4B facing the first syringe disposing recess 4A, a first lid recess 31A facing the first body recess 21A, a second lid recess 32A facing the second body recess 22A, a third lid recess 33A facing the third body recess 23A, a fourth lid recess 34A facing the fourth body recess 24A, and a fifth lid recess 35A facing the fifth body recess 25A.

The drive motor 21 is disposed between the first body recess 21A of the device body 2 and the first lid recess 31A of the lid 3 and serves as the drive body configured to generate drive power causing rotation of an operation part 44 to be described later, provided at the syringe 4. The output shaft 211 is disposed coaxially with the drive motor 21. The power source 26 supplies the drive motor 21 with electric power to rotationally drive the output shaft 211. The output shaft 211 accordingly generates rotational drive power causing rotation of the operation part 44 of the syringe 4. The power source 26 can be a secondary battery exemplified by a nickel-hydrogen storage battery, a nickel-cadmium storage battery, a lithium ion secondary battery, or a lithium ion polymer secondary battery.

The encoder 25 surrounds the output shaft 211 of the drive motor 21 and is disposed between the fifth body recess 25A of the device body 2 and the fifth lid recess 35A of the lid 3. The encoder 25 transmits, to the controller 27, a signal according to rotational speed of the output shaft 211. The controller 27 carries out drive control of the drive motor 21 in accordance with the signal transmitted from the encoder 25, to adjust the rotational speed of the output shaft 211 to a predetermined level.

The drive shaft 23 is disposed coaxially with the output shaft 211, between the third body recess 23A of the device body 2 and the third lid recess 33A of the lid 3. The drive shaft 23 is coupled to the output shaft 211 via the coupling 22, and rotates along with rotation of the output shaft 211. The coupling 22 is disposed between the second body recess 22A of the device body 2 and the second lid recess 32A of the lid 3, and serves as a shaft joint for transferring the rotational drive power of the output shaft 211 to the drive shaft 23.

The drive gear 24 is disposed between the fourth body recess 24A of the device body 2 and the fourth lid recess 34A of the lid 3, and is fixed to the drive shaft 23. The drive gear 24 is engaged with a gear part 442 to be described later, provided on an outer circumferential surface of the operation part 44 of the syringe 4, and transfers, to the operation part 44, rotational drive power of the drive shaft 23 rotating along with rotation of the output shaft 211.

The drive power transfer part in the syringe pump device 1 according to the present embodiment is configured by the coupling 22, the drive shaft 23, and the drive gear 24, and transfers the drive power generated by the drive motor 21 to the operation part 44 of the syringe 4.

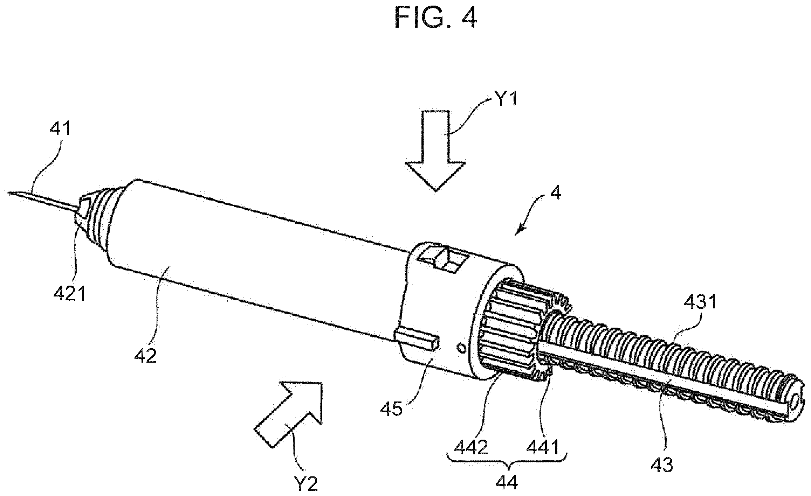

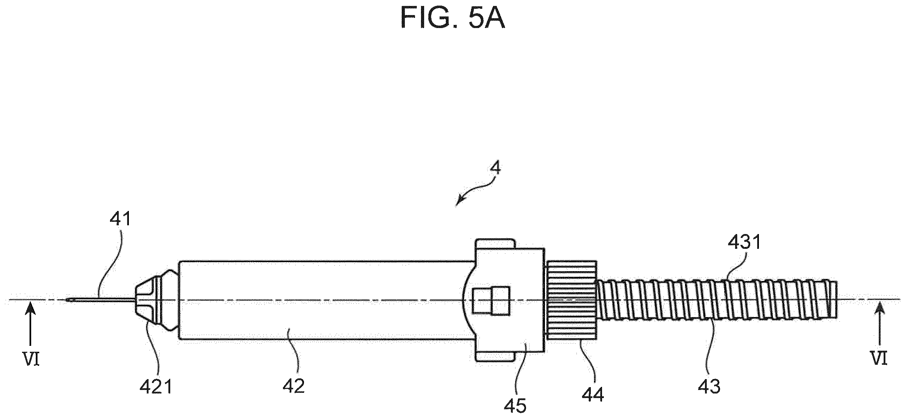

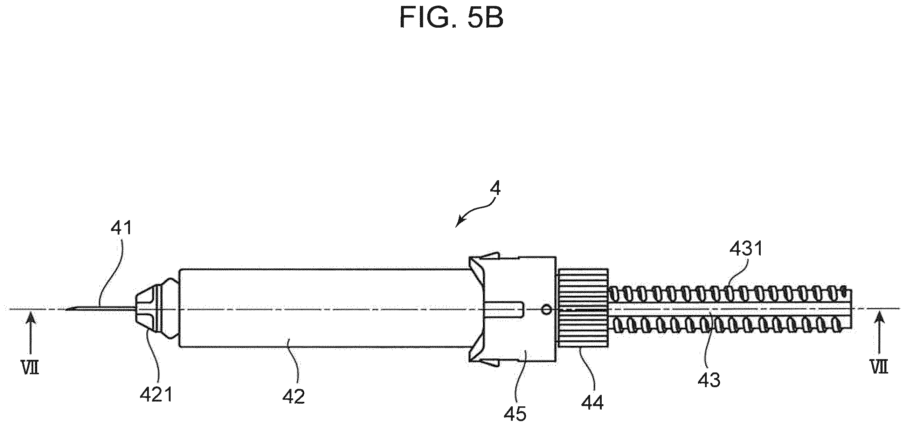

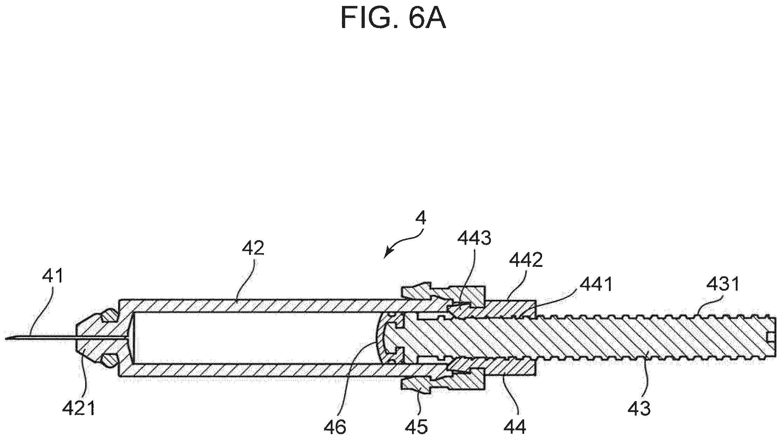

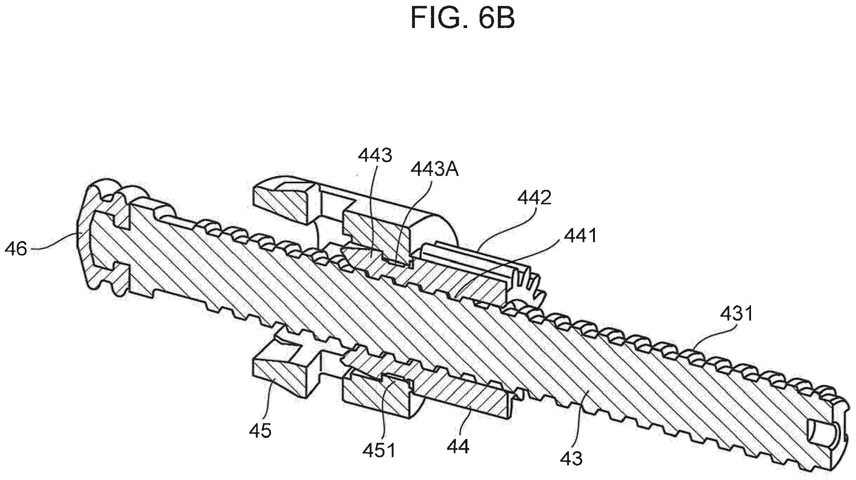

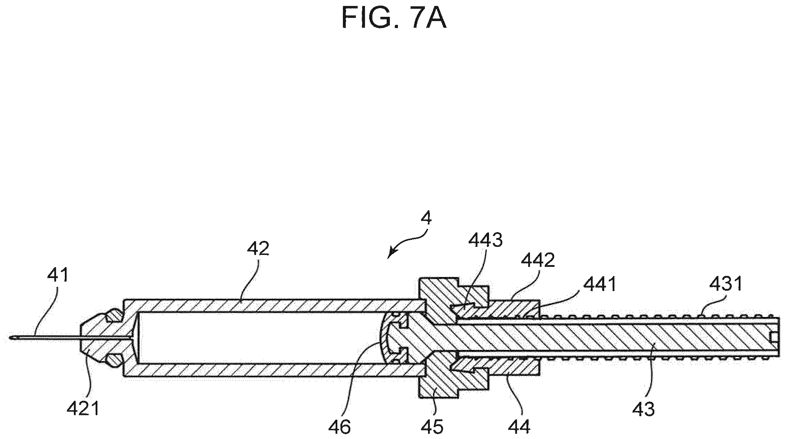

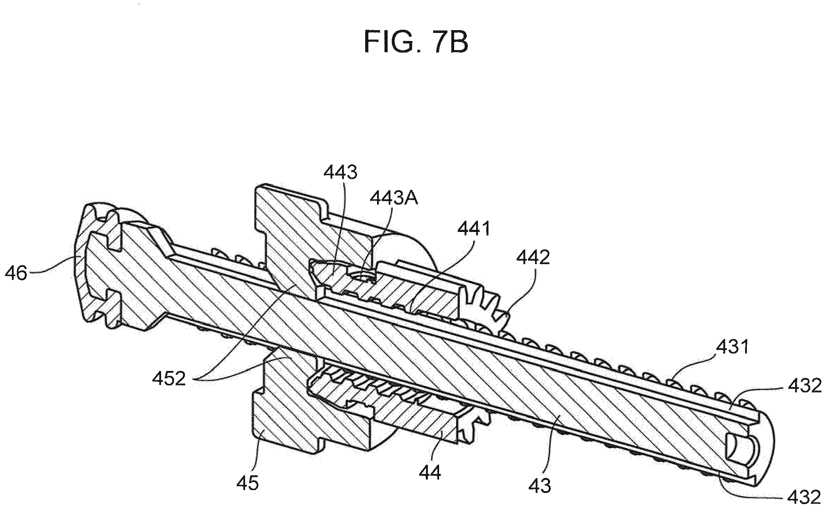

The syringe 4 is disposed between the first syringe disposing recess 4A of the device body 2 and the second syringe disposing recess 4B of the lid 3. The syringe 4 functions as a member configured to suck or discharge fluid in the syringe pump device 1. The syringe 4 is described below in terms of a configuration thereof with reference to FIGS. 4, 5A, 5B, 6A, 6B, 7A, and 7B. FIG. 4 is a perspective view showing the configuration of the syringe 4 according to an embodiment of the present invention. FIG. 5A is a plan view in a direction of an arrow Y1 indicated in FIG. 4, of the syringe 4. FIG. 5B is a side view in a direction of an arrow Y2 indicated in FIG. 4, of the syringe 4. FIG. 6A is a sectional view along a sectional line VI-VI, of the syringe 4 shown in FIG. 5A. FIG. 6B is a sectional view along the sectional line VI-VI, of part of the syringe 4 shown in FIG. 5A. FIG. 7A is a sectional view along a sectional line VII-VII, of the syringe 4 shown in FIG. 5B. FIG. 7B is a sectional view along the sectional line VII-VII, of part of the syringe 4 shown in FIG. 5B.

The syringe 4 includes a fluid flow-through channel body 41, a hub 421, a cylinder body 42, a plunger 43, the operation part 44, a support 45, and a gasket 46. In the present embodiment, the fluid flow-through channel body 41 and the hub 421 configure a fluid flow-through channel, the support 45 configures part of the cylinder body 42, and the fluid flow-through channel body 41, the hub 421, and the cylinder body 42 configure a cylinder.

The cylinder body 42 has a cylindrical shape provided with an internal space containing fluid, and has a first axial end connected with the hub 421 having a gradually decreased diameter. The fluid flow-through channel body 41 is coupled to the hub 421 connected to the first axial end of the cylinder body 42, and is provided with the fluid flow-through channel communicating with the internal space. The fluid flow-through channel body 41 corresponds to a syringe needle linearly extending axially outward from the hub 421 connected to the first axial end of the cylinder body 42. In the state where the syringe 4 is disposed between the first syringe disposing recess 4A of the device body 2 and the second syringe disposing recess 4B of the lid 3, the fluid flow-through channel body 41 has a distal end that is opposite to an end coupled to the hub 421 and projects outward from outer peripheral edges of the device body 2 and the lid 3.

The plunger 43 has a columnar shape and axially reciprocates in the cylinder 42. The plunger 43 has a first axial end provided with the gasket 46. The gasket 46 seals the cylinder 42 to achieve airtightness and fluidtightness of the internal space in a state where the plunger 43 is inserted to the cylinder 42. The plunger 43 of the syringe 4 according to the present embodiment has the outer circumferential surface provided with a spiral male screw 431, and a plunger groove 432 (first groove) linearly extending axially.

The operation part 44 has a tubular shape allowing the plunger 43 to be inserted therethrough, and is rotatable about an axis of the plunger 43 relative to the cylinder body 42. The operation part 44 has an inner circumferential surface provided with a spiral female screw 441 to be screwed to the male screw 431 of the plunger 43, and an outer circumferential surface provided with the gear part 442 receiving drive power causing axial rotation about the plunger 43. The gear part 442 of the operation part 44 is engaged with the drive gear 24 fixed to the drive shaft 23, so that the operation part 44 receives the rotational drive power of the drive shaft 23 rotating along with rotation of the output shaft 211 of the drive motor 21. When the rotational drive power of the drive shaft 23 is transferred to the operation part 44 via the gear part 442, the operation part 44 rotates about an axis of the plunger 43 relative to the cylinder body 42. The male screw 431 of the plunger 43 and the female screw 441 of the operation part 44 configure a transfer mechanism that transfers torque of the operation part 44 to the plunger 43, so that the torque of the operation part 44 is transferred to the plunger 43 via the male screw 431 and the female screw 441.

The operation part 44 includes an operation insert 443 inserted to the support 45 to be described later, provided at a second axial end of the cylinder body 42. The operation insert 443 causes the operation part 44 to be supported by the support 45 at the second axial end of the cylinder body 42. The operation insert 443 of the operation part 44 has an outer circumferential surface provided with an operation groove 443A (second groove) extending entirely circumferentially.

The support 45 has a tubular shape, is provided at the second axial end of the cylinder body 42, opposite to the end connected with the hub 421, and rotatably supports the operation part 44. The support 45 can be provided integrally with the second axial end of the cylinder body 42, or can be provided separately from the cylinder body 42. In the case where the support 45 is provided separately from the cylinder body 42, the support 45 has the tubular shape, is fixed to the second axial end of the cylinder 42, and rotatably supports the operation part 44. The support 45 at the second axial end of the cylinder body 42 includes an operation part shift restrictor 451 (second insert) shown in FIG. 6B, which is inserted to the operation groove 443A of the operation part 44 and restricts an axial shift of the operation part 44, and a plunger rotation restrictor 452 (first insert) shown in FIG. 7B, which is inserted to the plunger groove 432 of the plunger 43 and restricts rotation of the plunger 43 in the axis direction.

The operation groove 443A of the operation part 44 and the operation part shift restrictor 451 of the support 45 configure an operation part shift restrictive mechanism that restricts a shift along the axial direction of the plunger 43, of the rotated operation part 44. In the state where the operation part shift restrictor 451 of the support 45 is inserted to the operation groove 443A, the operation part 44 is restricted in terms of an axial shift, and is rotatable about an axis of the plunger 43. The plunger groove 432 of the plunger 43 and the plunger rotation restrictor 452 of the support 45 configure a plunger rotation restrictive mechanism that restricts rotation of the plunger 43 in the axis direction by receiving the torque of the operation part 44 via the male screw 431 and the female screw 441. In the state where the plunger rotation restrictor 452 of the support 45 is inserted to the plunger groove 432, the plunger 43 is restricted in terms of axial rotation but is axially reciprocatable.

The syringe pump device 1 according to the present embodiment has a converter including the transfer mechanism configured by the male screw 431 of the plunger 43 and the female screw 441 of the operation part 44, the operation part shift restrictive mechanism configured by the operation groove 443A of the operation part 44 and the operation part shift restrictor 451 of the support 45, and the plunger rotation restrictive mechanism configured by the plunger groove 432 of the plunger 43 and the plunger rotation restrictor 452 of the support 45. This converter converts the torque of the operation part 44 to axially reciprocating force of the plunger 43, and restricts a shift of the plunger 43 in an axial direction due to axial force applied to the plunger 43.

In the state where the operation part shift restrictor 451 is inserted to the operation groove 443A to restrict an axial shift of the operation part 44 in the syringe 4 configured as described above, when the operation part 44 receives drive power transferred via the gear part 442 and is rotated in a predetermined first rotation direction (e.g. counterclockwise), the male screw 431 engaged with the female screw 441 causes the plunger 43, which is restricted from rotating by the plunger rotation restrictor 452, to shift so as to project from the cylinder 42. This configuration thus allows fluid to be sucked from the fluid flow-through channel body 41 into the cylinder body 42. In the syringe 4 including the cylinder body 42 filled with the fluid, when the operation part 44 receives drive power transferred via the gear part 442 and is rotated in a second rotation direction (e.g. clockwise) reverse to the first rotation direction, the male screw 431 engaged with the female screw 441 causes the plunger 43, which is restricted from rotating by the plunger rotation restrictor 452, to shift so as to be accommodated in the cylinder body 42. The fluid can thus be discharged from the fluid flow-through channel body 41.

When the fluid is sucked into or discharged from the cylinder body 42 in the syringe 4 according to the present embodiment, the converter converts axial torque of the operation part 44 supported by the support 45 to reciprocating force of the plunger 43. Such a configuration enables precise control of a flow rate of the fluid sucked or discharged by the syringe 4. The converter restricts a shift of the plunger 43 in an axial direction due to axial force applied to the plunger 43. Even when the plunger 43 receives undesired axial force, the plunger 43 will not axially shift unless the operation part 44 is rotated, and restrains undesired suck or discharge of the fluid.

Furthermore, the converter in the syringe 4 according to the present embodiment includes the transfer mechanism configured by the male screw 431 of the plunger 43 and the female screw 441 of the operation part 44, and the operation part shift restrictive mechanism configured by the operation groove 443A of the operation part 44 and the operation part shift restrictor 451 of the support 45. In the syringe 4 thus configured, the torque of the operation part 44 is transferred to the plunger 43 via the male screw 431 and the female screw 441 and is converted to reciprocating force of the plunger 43. An axial shift of the operation part 44 is restricted by the operation part shift restrictive mechanism including the operation groove 443A and the operation part shift restrictor 451, so that the torque of the operation part 44 is kept transferred to the plunger 43 highly efficiently.

Moreover, the converter in the syringe 4 according to the present embodiment includes the plunger rotation restrictive mechanism configured by the plunger groove 432 of the plunger 43 and the plunger rotation restrictor 452 of the support 45. When the torque of the operation part 44 is transferred to the plunger 43 via the male screw 431 and the female screw 441 in the syringe 4 thus configured, rotation of the plunger 43 in the axis direction is restricted by the plunger rotation restrictive mechanism including the plunger groove 432 and the plunger rotation restrictor 452. Even when the plunger 43 receives undesired force causing axial rotation, the plunger 43 will not shift axially and restrains undesired suck or discharge of the fluid. This plunger rotation restrictive mechanism improves a sealing property of the plunger 43 to the cylinder body 42.

The syringe pump device 1 according to the present embodiment includes the syringe 4 configured as described above, to enable precise control of a flow rate of the sucked or discharged fluid, and restrain undesired suck or discharge of the fluid.

As shown in FIG. 2, the components of the syringe pump device 1, namely, the syringe 4, the drive motor 21, the output shaft 211, the coupling 22, the drive shaft 23, the drive gear 24, and the encoder 25 are interposed to be held between the device body 2 and the lid 3. In the state where the gear part 442 in the operation part 44 of the syringe 4 is engaged with the drive gear 24, the plunger 43 and the output shaft 211 are disposed parallelly to each other to be spaced in a widthwise direction perpendicularly intersecting the axial direction. The plunger 43 is positioned in an area of width of the drive motor 21, and is disposed to be axially separate by a predetermined distance from the drive motor 21 when most projected from the cylinder body 42. The syringe pump device 1 is thus made small in size with minimization in axial length and width.

The specific embodiment described above mainly includes the following novel configurations.

A syringe according to an aspect of the present invention includes: a cylinder including a cylinder body having an internal space to accommodate fluid, and a fluid flow-through channel part connected to a first axial end of the cylinder body and provided with a fluid flow-through channel communicating with the internal space; a plunger configured to axially reciprocate inside the cylinder body; an operation part having a tubular shape allowing the plunger to be inserted therethrough, and supported at a second axial end opposite to the first axial end of the cylinder body to be rotatable about an axis of the plunger relative to the cylinder body; and a converter configured to convert torque of the operation part to axially reciprocating force of the plunger, and to restrict a shift of the plunger in an axial direction due to axial force applied the plunger.

When the fluid is sucked into or discharged from the cylinder body in this syringe, the converter converts the axial torque of the operation part to the reciprocating force of the plunger. Such a configuration achieves precise control of a flow rate of the fluid sucked or discharged by the syringe. The converter restricts the axial shift of the plunger due to the axial force applied to the plunger. Even when the plunger receives undesired axial force, the plunger will not axially shift unless the operation part is rotated, and restrains undesired suck or discharge of the fluid.

In the syringe described above, the converter preferably includes a transfer mechanism configured to transfer the torque of the operation part to the plunger, and an operation part shift restrictive mechanism configured to restrict a shift along the axial direction of the plunger, of the rotated operation part.

In this syringe, the torque of the operation part is transferred to the plunger and is converted to the reciprocating force of the plunger. The axial shift of the operation part is restricted by the operation part shift restrictive mechanism, so that the torque of the operation part is kept transferred to the plunger highly efficiently.

In the syringe described above, the converter preferably further includes a plunger rotation restrictive mechanism configured to restrict rotation of the plunger in the axis direction by receiving the torque of the operation part transferred by the transfer mechanism.

When the torque of the operation part is transferred to the plunger by the transfer mechanism in the syringe, the rotation of the plunger in the axis direction is restricted by the plunger rotation restrictive mechanism. Even when the plunger receives undesired force causing axial rotation, the plunger will not shift axially and restrains undesired suck or discharge of the fluid. This plunger rotation restrictive mechanism improves a sealing property of the plunger to the cylinder body.

In the syringe described above, preferably, the plunger has an outer circumferential surface provided with a male screw and a first groove extending axially, the operation part has an inner circumferential surface provided with a female screw to be screwed to the male screw, and an outer circumferential surface provided with a second groove extending entirely circumferentially, the second axial end of the cylinder body is provided with a first insert inserted to the first groove and a second insert inserted to the second groove, the transfer mechanism comprises the male screw and the female screw, the plunger rotation restrictive mechanism comprises the first groove and the first insert, and the operation part shift restrictive mechanism comprises the second groove and the second insert.

This syringe includes the transfer mechanism simply configured by the male screw of the plunger and the female screw of the operation part, the plunger rotation restrictive mechanism simply configured by the first groove of the plunger and the first insert at the second axial end of the cylinder body, and the operation part shift restrictive mechanism simply configured by the second groove of the operation part and the second insert at the second axial end of the cylinder body.

In the syringe described above, the operation part preferably has the outer circumferential surface provided with a gear part receiving drive power causing rotation of the operation part.

This syringe is configured to transfer the drive power causing rotation of the operation part via the gear part.

A syringe pump device according to another aspect of the present invention includes: the syringe described above; a drive source configured to generate drive power causing rotation of the operation part in the syringe; a drive power transfer part configured to transfer the drive power generated by the drive source to the gear part of the operation part; and a holder holding the syringe, the drive source, and the drive power transfer part.

This syringe pump device enables precise control of a flow rate of the sucked or discharged fluid, and restrains undesired suck or discharge of the fluid.

In the syringe pump device described above, preferably, the drive source includes a drive body configured to generate drive power, and an output shaft disposed coaxially with the drive body, the drive power transfer part includes a drive shaft coupled coaxially with the output shaft, and a drive gear fixed to the drive shaft and engaged with the gear part of the operation part, in a state where the holder holds the syringe, the drive source, and the drive power transfer part, and the gear part is engaged with the drive gear, the plunger and the output shaft are disposed parallelly to each other to be spaced in a widthwise direction perpendicularly intersecting the axial direction, and the plunger is positioned in an area of width of the drive body, and is disposed to be separate by a predetermined distance from the drive body in the axial direction when most projected from the cylinder body.

This syringe pump device is thus made small in size with minimization in axial length and width.

As described above, the present invention provides such a syringe configured to suck or discharge fluid, precisely control a flow rate of the sucked or discharged fluid, and restrain undesired suck or discharged of the fluid, as well as provides a syringe pump device including the syringe.

## Claims

1. A syringe comprising:
a cylinder including a cylinder body having an internal space to accommodate fluid, and a fluid flow-through channel part connected to a first axial end of the cylinder body and provided with a fluid flow-through channel communicating with the internal space;
a plunger configured to axially reciprocate inside the cylinder body;
an operation part having a tubular shape allowing the plunger to be inserted therethrough, and supported at a second axial end opposite to the first axial end of the cylinder body to be rotatable about an axis of the plunger relative to the cylinder body; and
a converter configured to convert torque of the operation part to axially reciprocating force of the plunger, and to restrict a shift of the plunger in an axial direction due to axial force applied the plunger.

2. The syringe according to claim 1, wherein
the converter includes
a transfer mechanism configured to transfer the torque of the operation part to the plunger, and
an operation part shift restrictive mechanism configured to restrict a shift along the axial direction of the plunger, of the rotated operation part.

3. The syringe according to claim 2, wherein the converter further includes a plunger rotation restrictive mechanism configured to restrict rotation of the plunger in the axis direction by receiving the torque of the operation part transferred by the transfer mechanism.

4. The syringe according to claim 3, wherein
the plunger has an outer circumferential surface provided with a male screw and a first groove extending axially,
the operation part has an inner circumferential surface provided with a female screw to be screwed to the male screw, and an outer circumferential surface provided with a second groove extending entirely circumferentially,
the second axial end of the cylinder body is provided with a first insert inserted to the first groove and a second insert inserted to the second groove,
the transfer mechanism comprises the male screw and the female screw, the plunger rotation restrictive mechanism comprises the first groove and the first insert, and the operation part shift restrictive mechanism comprises the second groove and the second insert.

5. The syringe according to claim 4, wherein the operation part has the outer circumferential surface provided with a gear part receiving drive power causing rotation of the operation part.

6. A syringe pump device comprising:
the syringe according to claim 5;
a drive source configured to generate drive power causing rotation of the operation part in the syringe;
a drive power transfer part configured to transfer the drive power generated by the drive source to the gear part of the operation part; and
a holder holding the syringe, the drive source, and the drive power transfer part.

7. The syringe pump device according to claim 6, wherein
the drive source includes a drive body configured to generate drive power, and an output shaft disposed coaxially with the drive body,
the drive power transfer part includes a drive shaft coupled coaxially with the output shaft, and a drive gear fixed to the drive shaft and engaged with the gear part of the operation part,
in a state where the holder holds the syringe, the drive source, and the drive power transfer part, and the gear part is engaged with the drive gear,
the plunger and the output shaft are disposed parallelly to each other to be spaced in a widthwise direction perpendicularly intersecting the axial direction, and
the plunger is positioned in an area of width of the drive body, and is disposed to be separate by a predetermined distance from the drive body in the axial direction when most projected from the cylinder body.
